## Europäisches Patentamt
## European Patent Office
## Office européen des brevets

(19)

(11) Veröffentlichungsnummer: **0 297 250 B1**

(12)

# EUROPÄISCHE PATENTSCHRIFT

(45) Veröffentlichungstag der Patentschrift :
31.07.91 Patentblatt 91/31

(51) Int. Cl.⁵ : **A61F 2/38**

(21) Anmeldenummer : **88107139.3**

(22) Anmeldetag : **04.05.88**

(54) Metallener tibialer Verankerungsteil einer Kniegelenk-Teilprothese.

(30) Priorität : 30.06.87 CH 2462/87

(43) Veröffentlichungstag der Anmeldung :
04.01.89 Patentblatt 89/01

(45) Bekanntmachung des Hinweises auf die
Patenterteilung :
31.07.91 Patentblatt 91/31

(84) Benannte Vertragsstaaten :
AT DE FR GB IT

(56) Entgegenhaltungen :
FR-A- 2 550 936
GB-A- 1 390 494
US-A- 3 869 731

(73) Patentinhaber : GEBRÜDER SULZER
AKTIENGESELLSCHAFT
Zürcherstrasse 9
CH-8401 Winterthur (CH)
Patentinhaber : Protek AG
Stadtbachstrasse 64
CH-3001 Bern (CH)

(72) Erfinder : Noesberger, Bruno, Dr. med.
Regionalspital
CH-3800 Interlaken (CH)
Erfinder : Frey, Otto
Wallrütistrasse 56
CH-8400 Winterthur (CH)

## Beschreibung

Die Erfindung betrifft einen metallenen tibialen Verankerungsteil einer Kniegelenk-Teilprothese für eine zementfreie Verankerung im Tibiaknochen, wobei der Verankerungsteil einen Kunststoffkörper mit einer Gleit- und Lagerfläche für eine mediale oder laterale Femurkondyle aufnimmt.

Eine zementfreie Verankerung von Tibiateilen in der Tibia erfolgt bisher mit Hilfe von Zapfen oder Dübeln (EP-A-151724 und EP-A-170779), wobei diese Zapfen oder Dübel in Bohrungen im Tibiakopf eingebracht werden, die sich in Richtung der Tibia-Längsachse in den Knochen hinein erstrecken.

Bei nur wenig zerstörten, natürlichen Gelenken, bei denen der Bandapparat (Seiten- und Kreuzbänder) noch gesund und funktionsfähig ist, ergeben sich bei der operativen Verankerung der genannten Tibiateile Schwierigkeiten, da bei funktionsfähigem Bandapparat Femur und Tibia nur relativ geringfügig gegeneinander verschoben und in Richtung der Längsachse "auseinandergezogen" werden können.

Es ist daher schwierig, die für die geschilderten Verankerungen notwendigen Bohrungen in den Tibiakopf einzubringen, wenn der Bandapparat auch intraoperativ nicht gelöst werden soll.

Aufgabe der Erfindung ist es daher, einen Verankerungsteil für eine zementfreie Verankerung einer Tibia-Teilprothese zu schaffen, der auf dem Tibiakopf operativ verankert werden kann, ohne dass Eingriffe am Bandapparat notwendig sind.

Diese Aufgabe wird mit der Erfindung dadurch gelöst, dass am Tibia-Plateau eines ebenen und mindestens annähernd halbkreisförmigen Lagerelementes ein Fixierungselement angesetzt ist, das von ventral nach dorsal in Richtung distal abfallend verläuft, und dessen dorsale Vorderkanten als Schneiden ausgebildet sind.

Der neue Verankerungsteil ist von ventral nach dorsal in den Tibiakopf einschlagbar, dessen geschädigte —mediale oder laterale— Kondyle zuvor entfernt worden ist. Die Schnittfläche an der geräumten Kondyle des natürlichen Tibiakopfes bildet dann die Auflagefläche für das Tibia-Plateau des Verankerungsteils.

Das von ventral nach dorsal in Richtung distal schräg abfallende Fixierungselement, das mit Vorteil einen blattartigen Stamm mit einer Verbreiterung am distalen Ende aufweist, verhindert ein schubladenartiges Herausschieben des Implantates aus dem Knochen.

Eine vorteilhafte Konstruktion für das Fixierungselement ergibt sich dabei, wenn das Fixierungselement T-förmig ausgebildet ist, wobei dessen vertikaler Stamm eine von zwei Seiten zur Mitte hin verlaufende Schneide aufweist, während die Schneidkanten des Querbalkens einseitig nach distal angeschärft ist ; die Form der vertikalen Schneide bewirkt eine Verteilung des verdrängten Knochengewebes nach beiden Seiten, während mit der einseitigen Schneide des Querbalkens eine Verdichtung der verdrängten Spongiosa in Richtung proximal erreicht wird, wodurch die Haftung des Implantates im Knochen gefördert wird. Diese Haftung kann langfristig weiter verbessert werden, wenn das den Stamm bildende Blatt mit Durchbrüchen versehen ist.

Schliesslich ist es zur Anpassung an die anatomischen Verhältnisse vorteilhaft, wenn die vertikalen Kanten des Stammes nach ventral geneigt und/oder das Lagerelement gegen die Horizontale nach distal/dorsal geneigt sind.

Der neue Verankerungsteil besteht aus einem der bekannten metallischen Prothesenwerkstoffe. Er kann geschmiedet oder gegossen sein. Darüberhinaus können seine Kontaktflächen zum Knochen in bekannter Weise mit einer Beschichtung und/oder Struktur versehen sein.

Die nicht gezeigte künstliche Tibiakondyle wird als Gleit- und Lagerfläche gegenüber einer ebenfalls vorzugsweise metallischen Femurprothese beispielsweise aus Polyäthylen der für Implantate üblichen Spezifikationen hergestellt. Sie kann sowohl bereits während der Fabrikation mit dem Verankerungsteil verbunden werden als auch— mit Hilfe von geeigneten Verschlüssen— erst intraoperativ auf dem Verankerungsteil befestigt werden.

Im folgenden wird die Erfindung anhand von Ausführungsbeispielen im Zusammenhang mit der Zeichnung näher erläutert.

| | |
|---|---|
| Fig. 1 | ist schematisch eine Aufsicht auf das Tibia-Plateau des neuen Verankerungsteils ; |
| Fig. 2 | ist eine Ansicht von Fig. 1 von oben ; |
| Fig. 3 | gibt eine Ansicht von Fig. 2 von links wieder ; |
| Fig. 4 und 5 | sind Ansichten in Richtung lateral bzw. dorsal eines Tibiakopfes mit eingesetztem Implantat nach Fig. 1 bis 3 ; |
| Fig. 6 bis 10 | schliesslich geben verschiedene Profile für das Fixierungselement wieder. |

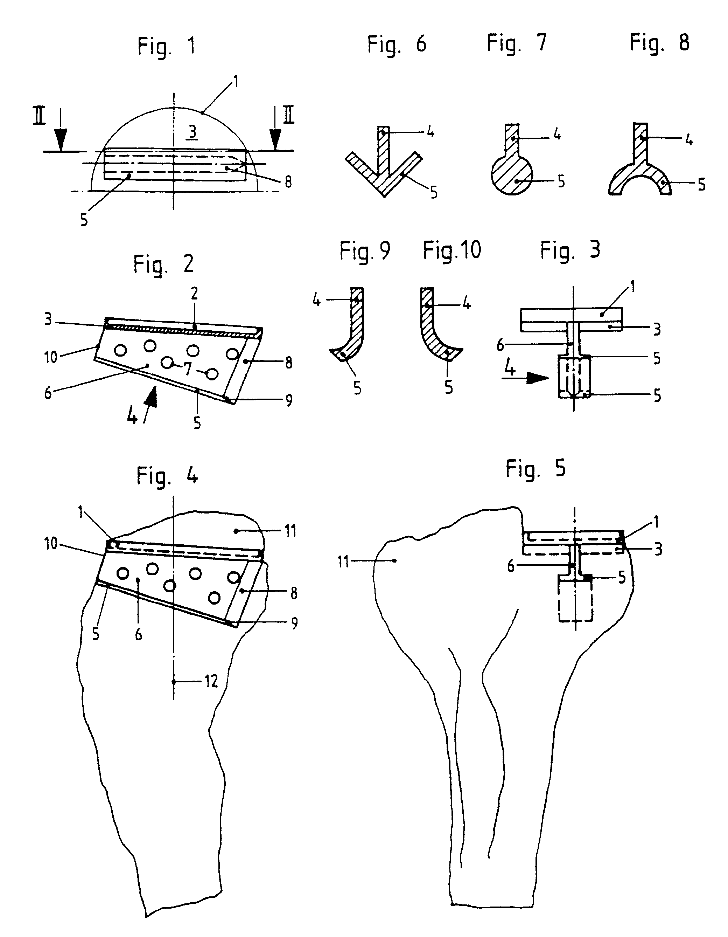

An das Tibia-Plateau 3 eines im Grundriss halbkreisförmigen Lagerelementes 1 (Fig. 1), das in seiner oberseitigen Kondylenfläche eine schematisch angedeutete Vertiefung 1 (Fig. 2) für die Aufnahme eines nicht gezeigten Kunststoffkörpers aufweist, ist als Fixierungselement 4 ein T-Stück mit seiner Verbreiterung, d.h. seinem Querbalken 5 nach distal gerichtet, angesetzt. Der blattartige Stamm 6 des T-Stückes, der mit Durchbrü-

EP 0 297 250 B1

chen 7 versehen ist, ist auf seiner nach dorsal gerichteten "Vorderseite" mit einer doppelseitigen Schneidkante 8 versehen. Ebenso weist der Querbalken 5 eine Schneidkante 9 (Fig. 2) auf, die jedoch einseitig nach proximal gerichtet ist, so dass von ihr gelöste Spongiosa nach "oben" verdrängt wird.

Erfindungsgemäss verbreitert sich die Fläche des Stammes 6, dessen Vorderkante 8 und dessen Hinterkante 10 zur Anpassung an die anatomischen Verhältnisse nach ventral geneigt sind, in Richtung ventral/dorsal, so dass der Querbalken 5 von ventral nach dorsal in einem Winkel von etwa 17° gegen die Horizontale nach distal geneigt verläuft. Diese Neigung, die generell zwischen 15° und 20° gegen die Horizontale betragen kann, bildet für den in einen Tibiakopf 11 (Fig. 5 und 6) eingeschlagenen Verankerungsteil eine Sicherung gegen ein ungewolltes Herausschieben.

Darüberhinaus ist bei dem in den Fig. 1 bis 5 gezeigten Ausführungsbeispiel zusätzlich auch das Lagerelement 1 gegen die Tibiaachse 12 (Fig. 5) bzw. die dazu senkrechte Horizontale um wenige Grad — beispielsweise bis zu 5° — nach dorsal/distal geneigt. Diese Neigung, die jedoch gegebenenfalls entbehrlich ist, bewirkt, dass die resultierende Belastung eine geringe horizontale Kraftkomponente in Richtung dorsal hat, die die Sicherheit gegen ein Herausschieben des Implantats zusätzlich vergrössert.

Wie erwähnt, erfolgt die Implantation des Verankerungsteils durch einfaches Einschlagen des Implantates in den entsprechend vorbereiteten Tibiakopf 11, wobei die Schneidkanten 8 und 9 das relativ weiche spongiose Gewebe verdrängen.

In den Fig. 6 bis 10 sind weitere Querschnittsformen für blattartige Fixierungselemente 4 mit unterschiedlichen Verbreiterungen 5 gezeigt.

## Patentansprüche

1. Metallener tibialer Verankerungsteil einer Kniegelenk-Teilprothese für eine zementfreie Verankerung im Tibiaknochen, wobei der Verankerungsteil einen Kunststoffkörper mit einer Gleit- und Lagerfläche für eine mediale oder laterale Femurkondyle aufnimmt, dadurch gekennzeichnet, dass am Tibia-Plateau (3) eines ebenen und mindestens annähernd halbkreisförmigen Lagerelementes (1) ein Fixierungselement (4) angesetzt ist, das von ventral nach dorsal in Richtung distal abfallend verläuft, und dessen dorsale Vorderkanten als Schneiden (8 und 9) ausgebildet sind.

2. Verankerungsteil nach Anspruch 1, dadurch gekennzeichnet, dass das Fixierungselement (4) einen blattartigen vertikalen Stamm (6) aufweist, der an seinem distalen Ende eine Verbreiterung (5) trägt.

3. Verankerungsteil nach Anspruch 1 oder 2, dadurch gekennzeichnet, dass das Fixierungselement (4) T-förmig ausgebildet ist, wobei dessen vertikaler Stamm (6) eine von zwei Seiten zur Mitte hin verlaufende Schneide (8) aufweist, während die Schneikante (9) des Querbalkens (5) einseitig nach distal angeschärft ist.

4. Verankerungsteil nach einem der Ansprüche 1 bis 3, dadurch gekennzeichnet, dass das den Stamm bildende Blatt mit Durchbrüchen (7) versehen ist.

5. Verankerungsteil nach einem der Ansprüche 1 bis 4, dadurch gekennzeichnet, dass die vertikalen Kanten (8, 10) des Stammes (6) nach ventral geneigt sind.

6. Verankerungsteil nach einem der Ansprüche 1 bis 5, dadurch gekennzeichnet, dass das Lagerelement (1) gegen die Horizontale nach distal/dorsal geneigt ist.

## Revendications

1. Pièce d'ancrage métallique tibiale d'une prothèse partielle de l'articulation du genou destinée à un ancrage sans ciment dans le tibia, la pièce d'ancrage supportant un corps de matière synthétique comportant une surface de glissement et d'appui d'un condyle médial ou latéral du fémur, caractérisée en ce qu'un élément de fixation (4) est rapporté sur le plateau du tibia (3) d'un élément d'appui (1) plan et au moins approximativement semi-circulaire, ledit élément de fixation étant en pente descendante du côté ventral vers le côté dorsal en direction distale et ses bord antérieurs dorsaux étant conformés en tranchants (8 et 9).

2. Pièce d'ancrage selon la revendication 1, caractérisée en ce que l'élément de fixation (4) comprend un tronc vertical (6) en forme de lame qui comporte un élargissement (5) à l'extrémité distale.

3. Pièce d'ancrage selon la revendication 1 ou 2, caractérisée en ce que l'élément de fixation (4) est conformé en T dont le tronc vertical (6) présente un tranchant (8) allant de deux côtés vers le milieu, tandis que l'arête tranchante (9) de la traverse (5) est affûtée d'un côté vers l'extrémité distale.

4. Pièce d'ancrage selon l'une des revendications 1 à 3, caractérisée en ce que la lame formant le tronc comporte des trous (7).

5. Pièce d'ancrage selon l'une des revendications 1 à 4, caractérisée en ce que les bords verticaux (8, 10)

3

du tronc (6) sont inclinés vers le côté ventral.

6. Pièce d'ancrage selon l'une des revendications 1 à 5, caractérisée en ce que l'élément d'appui (1) est incliné sur l'horizontale vers le côté distal/dorsal.

## Claims

1. A metal tibial fixing member of a knee joint partial prosthesis for uncemented fixing in the tibia, the fixing member receiving a plastics member having a sliding and bearing surface for a medial or lateral femoral condyle, characterised in that a fixing element (4) is secured to the tibia plateau (3) of a plane and at least substantially semicircular bearing element (1) and extends ventrally to dorsally with a descent in the distal direction, the dorsal front edges of the fixing element being cutting edges (8, 9).

2. A fixing member according to claim 1, characterised in that the fixing element (4) has a blade-like vertical stem (6) which has a widening (5) at its distal end.

3. A fixing member according to claim 1 or 2, characterised in that the fixing element (4) is T-shaped, its vertical stem (6) having a cutting edge (8) extending from two sides towards the centre while the cutting edge (9) of the transverse member (5) is sharpened unilaterally in the distal direction.

4. A fixing member according to any of claims 1 to 3, characterised in that the stem-forming blade is formed with apertures (7).

5. A fixing member according to any of claims 1 to 4, characterised in that the vertical edges (8, 10) of the stem (6) are inclined ventrally.

6. A fixing member according to any of claims 1 to 5, characterised in that the bearing element (1) is inclined to the horizontal distally to dorsally.

Fig. 1

Fig. 6    Fig. 7    Fig. 8

Fig. 2

Fig. 9    Fig.10    Fig. 3

Fig. 4

Fig. 5